(19) Europäisches Patentamt
European Patent Office
Office européen des brevets

(11) Veröffentlichungsnummer: **0 046 907**
**B1**

# (12) EUROPÄISCHE PATENTSCHRIFT

(45) Veröffentlichungstag der Patentschrift: **28.03.84**

(51) Int. Cl.³: **A 61 K 6/06,** A 61 K 6/08, A 61 K 6/10

(21) Anmeldenummer: **81106304.9**

(22) Anmeldetag: **13.08.81**

(54) **Vinylsilikon-Pasten für die Zahnabformung.**

(30) Priorität: **23.08.80 DE 3031894**

(43) Veröffentlichungstag der Anmeldung: **10.03.82 Patentblatt 82/10**

(45) Bekanntmachung des Hinweises auf die Patenterteilung: **28.03.84 Patentblatt 84/13**

(84) Benannte Vertragsstaaten: **AT BE CH DE FR GB IT LI LU NL SE**

(56) Entgegenhaltungen:
**DE - A - 2 926 405**
**FR - A - 2 262 955**
**FR - A - 2 400 052**

**ULLMANNS ENCYKLOPÄDIE DER TECHNISCHEN CHEMIE, 4. Auflage, Band 13, Verlag Chemie, 1977, Seiten 533-534 Weinheim, DE**

(73) Patentinhaber: **BAYER AG, Konzernverwaltung RP Patentabteilung, D-5090 Leverkusen 1 Bayerwerk (DE)**

(72) Erfinder: **Puppe, Lothar, Dr., Gisbert-Cremer-Strasse 45a, D-5090 Leverkusen 1 (DE)**
Erfinder: **Voigt, Reiner, Gustav-Radbruch-Strasse 14, D-5090 Leverkusen 3 (DE)**
Erfinder: **Borgardt, Manfred, Dr., Rödiger Strasse 17, D-5600 Wuppertal 2 (DE)**
Erfinder: **Moretto, Hans-Heinrich, Dr., Formesstrasse 13, D-5000 Köln 80 (DE)**
Erfinder: **Munchenbach, Bernard, Dr., Carl-Rumpff-Strasse 8, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Vinylsilikonpasten für die Zahnabformung

Die vorliegende Erfindung betrifft additionsvernetzende Vinylsilikonpasten zum Herstellen genauer Abformungen von Zähnen. Hierbei handelt es sich um ein kaltvulkanisierendes Zweikomponenten-Silikonkautschuksystem, bei dem eine vernetzerhaltige Basispaste mit einer Katalystatorpaste vermengt wird und nach 2-5 min bei Raumtemperatur vernetzt.

Silikonpasten zur Zahnabformung sind weitverbreitet. Im allgemeinen bestehen sie aus einem mit Füllstoffen vermischten Silikonöl auf Basis eines hydroxylendgestoppten Polydimethylsiloxans, wegen vieler Applikationsmethoden in verschiedenen Konsistenzen angeboten, und einer flüssigen oder pastenförmigen Härterkomponente, die ein Metallsalz einer Monocarbonsäure als Katalysator und einen Kieselsäureester als Vernetzer enthält.

Auch diese beiden Komponenten werden vor der Anwendung miteinander gemischt und vernetzen bei Raumtemperatur innerhalb von 2-5 min infolge einer Polykondensationsreaktion. Hierbei entstehen neben dem vernetzten Silikongummi noch geringe Mengen Alkohol, die langsam aus dem Gummi heraus diffundieren und eine lineare Schrumpfung hervorrufen, was bei den Abformung zu Ungenauigkeiten führt.

Wesentlich geringer ist die lineare Schrumpfung bei den Vinylsilikonabformmassen, deren Herstellung erst seit wenigen Jahren bekannt ist. Diese Massen bestehen aus zwei Pasten, einer Silikonöl, Füllstoff und Vernetzer enthaltenden Basispaste und einer Silikonöl, Füllstoff und Katalysator enthaltenden Katalysatorpaste.

Bei dem Silikonöl handelt es sich hierbei um ein vinylendgestopptes Polydimethylsiloxan, der Vernetzer enthält die reaktiven SiH-Gruppen und der Katalysator besteht aus einem Platinkomplex. Zu der grösseren Dimensionsgenauigkeit des Modells kommt bei diesem System noch die bessere Dosierbarkeit von Basis- und Katalysatorpaste infolge gleicher Pastenviskosität und dem abgestimmten Mischungsverhältnis von 1:1 der beiden Pasten hinzu sowie die völlige Geschmacks- und Geruchslosigkeit der Pasten.

Nachteilig bei den Vinylsilikonabformmassen ist die Entwicklung von Wasserstoffgas beim Ausgiessen der vernetzten Abformung mit einer Gipsaufschlämmung und die dadurch verursachte fehlerhafte Gipsmodelloberfläche. Dieses Wasserstoffgas bildet sich nach der Reaktion der Vinylgruppen des Silikonöles mit den SiH-Gruppen des Vernetzers aus den nicht verbrauchten, da im Überschuss vorhandenen SiH-Gruppen, begünstigt durch die Feuchtigkeit der Gipsaufschlämmung und den Platinkatalysator.

Die geschädigte Oberfläche des Gipsmodells weist eine Vielzahl von kleinen Kratern auf, so dass man das Modell als unbrauchbar bezeichnen muss. Eine deutliche Verbesserung der Situation tritt ein, wenn man die Abformung nach der Abdrucknahme wenigstens 2 h liegen lässt oder durch Erhitzen durch Unterdruck entgast, bevor man das Ausgiessen mit der Gipsaufschlämmung vornimmt. Jedoch sind diese Operationen sehr zeitaufwendig oder bedürfen besonderer Sorgfalt, wenn man keine Verschlechterung der Dimensionsgenauigkeit des Modells verursachen will.

In der DE-OS Nr. 2926405 wird ein Verfahren beschrieben, das den Versuch unternimmt, diese Nachteile zu vermeiden. Dabei wird Palladium oder Palladiumlegierung zu der Vernetzer- und/oder Katalysatorpaste zugegeben, um somit die bekannte Absorption von Wasserstoff an Palladium auszunutzen.

Demgegenüber bringt erfindungsgemäss der Einsatz von palladiumhaltigen Zeolith in der Vernetzer- und/oder Katalysatorpaste Vorteile. Das Palladium liegt in besonders fein verteilter Form vor, was sich äusserst günstig auf die Absorption von überschüssigen Wasserstoffgas auswirkt, ohne dass die Vernetzungsreaktion und die Lagerstabilität der Masse ungünstig beeinflusst wird. Der Einsatz von toxikologisch bedenklichen Metallstäuben wird vermieden. Ausserdem nimmt Zeolith Feuchtigkeitsspuren der Füllstoffe auf, so dass von dieser Seite zuverlässig eine zusätzliche Wasserstoffgaz-Entwicklung verhindert wird.

Problemlose Abhilfe dieses Mangels wird erfindungsgemäss also durch den Einsatz von palladiumhaltigem Zeolith in Vernetzer- und/oder Katalysatorpaste gegeben. In diesem Zeolith, welcher ausserdem noch Feuchtigkeitsspuren der Füllstoffe aufnehmen kann, ist das Palladium besonders fein verteilt, was sich äusserst günstig auf die Absorption von überschüssigem Wasserstoffgas auswirkt, ohne dass die Vernetzungsreaktion und die Lagerstabilität der Masse ungüstigt beeinflusst werden.

Gegenstand der Erfindung sind somit:

Bei Umgebungstemperatur härtbare Dentalmassen auf Polysiloxanbasis, die nach dem Additionssystem vernetzen, enthaltend

a) Organopolysiloxane mit zwei oder mehr Vinylgruppen im Molekül,

b) Organohydrogenpolysiloxan,

c) einem Katalysator zur Beschleunigung der Additionsreaktion,

d) hydrophobe Füllstoffe sowie gegebenenfalls weitere übliche Zusatzstoffe,

dadurch gekennzeichnet, dass den Massen zusätzlich

e) Alumosilicate, die fein verteiltes Palladium und/oder Palladiumlegierungen in feinster Verteilung enthalten,

zugesetzt werden.

So zeichnen sich die erfindungsgemässen Vinylsilikonpasten für die Herstellung genauer Abformungen von Zähnen durch ihre detailgetreue Wiedergabe des Gipsmodells aus, nachdem die Vernetzer- und Katalysatorpaste gut gemischt in die Mundhöle eingebracht und darin verfestigt wurde, die Abformung mit einer Gipsaufschlämmung ausgegossen und zu einem Modell ausgehärtet worden ist.

Die Einsatzstoffe der erfindungsgemässen Vinylsilikonpasten sind Silikonöl a, Füllstoffe b, Vernetzer c, Katalysator d, Farbstoffe e und schliesslich der palladiumhaltige Zeolith.

Bei dem Silikonöl a handelt es sich um ein vinylendgestopptes Polydimethylsiloxan, dessen Viskosität im Bereich von 1 000 bis 100 000 mPa·s bei 25° C liegen kann, je nach gewünschter Konsistenz der formulierten Pasten.

Unter den Füllstoffen b versteht man Quarz- und Cristobalitmehle, Calciumsulfat, Diatomeenerde, gefälltes und pyrogen hergestelltes Siliciumdioxid.

Der Vernetzer e ist ein Polydimethylsiloxan, welches in seinem Molekül Wasserstoffatome an mindestens zwei Siliciumatomen aufweist.

Die Siloxancopolymere mit unterschiedlichen Polymerisationsgrad, die durch Trialkylsilyl- oder Dialkylhydrogensilsubstanzengruppen endgestoppt sind, sind bekannt.

Bei dem Katalysator d handelt es sich um ein Platinkomplex, der aus Hexachloroplatin(IV)säure hergestellt wurde. Auch diese Verbindungen sind an sich bekannt.

Farbstoffe e werden zur Unterscheidung der Basis- und Katalisatorpaste und zur Mischkontrolle eingesetzt. Anorganische und organische Farbpigmente werden bevorzugt.

Die für die erfindungsgemässen Massen eingesetzten Zeolithe sind natürliche oder synthetische kristalline Aluminosilikate mit folgender allgemeinen oxidischen Formel:

$$X[(M', M''_{1/2}) \cdot AlO_2-] \cdot y\, SiO_2 \cdot zH_2O$$

mit M' = Li, Na, K usw.
M'' = Mg, Ca, Sr usw.

und beispielsweise beschrieben in D.W. Breck, „Zeolite Molekular Sieves", John Wiley & Sons, Inc., New York (1974).

Sie sind gekennzeichnet durch ein starres, dreidimensionales aus $SiO_4$- und $AlO_4$-Tetraedern bestehendes Netzwerk. Dieses Netzwerk lässt in seinem Innern grosse Adsorptionshohlräume frei, die untereinander durch Kanäle, den sogenannten Poren, verbunden sind. In den Adsorptionshohlräumen können polare oder polarisierbare Moleküle reversibel adsorbiert werden, deren Durchmesser geringer ist als der Porendurchmesser des betreffenden Zeoliths. Bevorzugt wird Wasser adsorbiert, weswegen Zeolithe hochwirksame Trokkenmittel sind.

Die im Gerüst vorhandenen $AlO_4$-Tetraeder verursachen jeweils eine negative Ladung, die durch Kationen ausgeglichen wird. Die im Zeolithe vorhandenen Kationen sind austauschbar, wobei man durch den Ionenaustausch die Porenweiten, die Adsorptionseigenschaften und das katalytische Verhalten entsprechend der Natur der ausgetauschten Metallkationen beeinflussen kann.

Einige Vertreter der Übergangsmetalle, die im Zeolith nach einem Ionenaustausch kationisch vorliegen, lassen sich mit Reduktionsmitteln zu den Metallen reduzieren. Hierbei erhält man hochdisperse Metalle in der kristallinen Aluminosilikatmatrix.

Die erfindungsgemässen Vinylsilikonpasten enthalten wasserfreie Zeolithe, die eine Faujasit-Struktur aufweisen und feinverteiltes metallisches Palladium besitzen. Synthetischer Faujasit hat die allgemeine Zusammensetzung:

$$(1{,}0 \pm 0{,}2)\ M_{\frac{2}{n}}O \cdot Al_2O_3 \cdot Y\, SiO_2$$

Synthetische Faujasite mit den Werten für y von 2 bis 3 werden allgemein als Zeolith X, solche mit Werten für y von 3 bis 6 als Zeolith Y bezeichnet.

Die Präparation palladiumhaltiger Zeolithe ist bereits an anderen Stellen beschrieben, z.B. H.S, Sherry, „The Ion Exchange", vol. 2, S. 89-133, New York (1969) und ist nicht Gegenstand der vorliegenden Erfindung.

Die eingesetzten Zeolithe enthalten im allgemeinen etwa 0,01 bis 5 Gew.-% Palladium bzw. Palladiumlegierung, bevorzugt 0,1 bis 2 Gew.-%. Der Paste werden insgesamt etwa 0,1 bis 5, bevorzugt etwa 0,3 bis 2 Gew.-% Zeolithe zugesetzt, so dass die Gesamtmasse etwa 0,5 bis 100 ppm, bevorzugt 5 bis 50 ppm Palladium bzw. Palladiumlegierung enthält.

Die nachfolgenden Beispiele, in denen alle Teile Gewichtsteile bedeuten, erläutern die Erfindung.

*Beispiel 1:*

Die Basispaste wurde hergestellt durch Vermischen in einem Kneter von 450 Teilen vinylendgestopptem Polydimethylsiloxan mit einer Viskosität von 10 000 mPa·s bei 25° C, 50 Teilen dimethylhydrogensilylendgestopptem Polydimethylsiloxan mit einer Viskosität von 50 mPa·s bei 25°C, 350 Teilen Quarzfeinstmehl, 125 Teilen Calciumsulfat, 20 Teilen pyrogen hergestellte Kieselsäure mit einer spez. Oberfläche von 50 $m^2/g$ nach BET und 5 Teile organischem Farbigment.

Die Herstellung der Katalysatorpaste erfolgte durch Vermischen in einem Kneter von 500 Teilen vinylendgestopptem Polydimethylsiloxan mit einer Viskosität von 10 000 mPa·s bei 25° C, 350 Teilen Quarzfeinstmehl, 125 Teilen Calciumsulfat, 24,8 Teilen pyrogen hergestellte Kieselsäure mit einer spez, Oberfläche von 50 $m^2/g$ nach BET und 0,2 Teilen Platin/Siloxan-Komplex.

15 g Basispaste und 15 g Katalysatorpaste wurden mit Hilfe einer Mischplatte 30 s gut vermischt, auf einen Abformlöffel und unter geeignetem Druck in den Mund gebracht. Innerhalb von 5 min härtet das Gemisch zu einem Elastomeren aus. Nach der Entnahme und Abwaschen wurde die Abformung mit einer Gipsaufschlämmung, hergestellt durch Vermischen von 100 Teilen Calciumsulfathemihydrat mit 30 Teilen Wasser, ausgegossen. 30 min später wurde das gebildete Gipsmodell aus der Abformung entnommen. Das Modell war von kleinen Kratern übersät und somit unbrauchbar.

*Beispiel 2:*

In einem Kneter wurde die Basispaste hergestellt durch Vermischen von 450 Teilen vinylendgestopptem Polydimethylsiloxan mit einer Viskosität von 10 000 mPa·s bei 25°C, 50 Teilen dimethylhydrogensilylendgestopptem Polydimethylsiloxan mit einer Viskosität von 50 mPa·s bei 25°C, 350 Teilen Quarzfeinstmehl, 120 Teilen Calciumsulfat, 20 Teilen pyrogen hergestellte Kieselsäure mit einer speziellen Oberfläche von 50 $m^2/g$ nach BET, 5 Teile organisches Farbpigment und 5 Teile Zeolith mit einem Palladiumgehalt von 0,2%.

Diese Basispaste wurde mit der Katalysatorpaste aus Beispiel 1 im Gewichtsverhältnis 1:1 gemischt, eine Abformung und danach ein Gipsmodell hergestellt wie in Beispiel 1.

Dieses Gipsmodell wies keinerlei Oberflächenschädigung auf.

*Beispiel 3:*

Die Katalysatorpaste wurde in einem Kneter hergestellt durch Vermischen von 500 Teilen vinylendgestopptem Polydimethylsiloxan mit einer Viskosität von 10 000 mPa·s bei 25°C, 350 Teilen Quarzfeinstmehl, 125 Teilen Calciumsulfat, 23,8 Teilen pyrogen hergestellte Kieselsäure mit einer spez. Oberfläche von 50 $m^2/g$ nach BET, 0,2 Teilen Platin/Siloxankomplex und 1 Teil Zeolith mit einem Palladiumgehalt von 1%.

Diese Katalysator wurde mit der Basispaste aus Beispiel 1 im Gewichtsverhältnis 1:1 gemischt, eine Abformung und danach ein Gipsmodell hergestellt wie in Beispiel 1.

Auch dieses Gipsmodell wies eine einwandfreie glatte Oberfläche auf.

*Beispiel 4:*

In einem Kneter wurde die Basispaste hergestellt durch Vermischen von 380 Teilen vinylgestopptem Polydimethylsiloxan mit einer Viskosität von 10 000 mPa·s bei 25°C, 70 Teilen vinylendgestopptem Polydimethylsiloxan mit einer Viskosität von 1000 mPa·s bei 25°C, 50 Teilen dimethylhydrogensilylendgestopptem Polydimethylsiloxan mit einer Viskosität von 50 mPa·s bei 25°C, 300 Teilen Quarzfeinstmehl, 190 Teilen Diatomeenerde, 5 Teilen organische Farbpigmente und 5 Teilen Zeolith mit 0,05% Palladiumgehalt.

Die Katalysatorpaste, hergestellt durch Mischen im Kneter, enthielt 400 Teile vinylendgestopptes Polydimethylsiloxan mit einer Viskosität von 10 000 mPa·s bei 25°C, 100 Teile vinylendgestopptes Polydimethylsiloxan mit einer Viskosität von 1000 mPa·s bei 25°C, 300 Teile Quarzfeinstmehl, 190 Teile Diatomeenerde, 0,2 Teile Platin/Siloxan-Komplex und 5 Teile Zeolith mit 0,05% Palladiumgehalt. Die beiden Pasten wurden im Gewichtsverhältnis 1:1 gemischt, eine Abformung und ein Gipsmodell angefertigt wie in Beispiel 1. Das Gipsmodell zeigte eine gleichförmige und glatte Oberfläche.

## Patentansprüche

1. Bei Umgebungstemperatur härtbare Dentalmassen auf Polysiloxanbasis, die nach dem Additionssystem vernetzen, enthaltend

a) Organopolysiloxane mit zwei oder mehr Vinylgruppe im Molekül,

b) Organohydrogenpolysiloxan,

c) einen Katalysator zur Beschleunigung der Additionsreaktion, und

d) hydrophobe Füllstoffe sowie gegebenenfalls weitere übliche Zusatzstoffe,

dadurch gekennzeichnet, dass den Massen zusätzlich

e) Alumosilicate, die fein verteiltes Palladium und/oder Palladiumlegierungen in feinster Verteilung enthalten, zugesetzt werden.

2. Massen nach Anspruch 1, dadurch gekennzeichnet, dass als Aluminosilicate Zeolithe vom Faujasittyp eingesetzt werden.

3. Massen nach einem der Ansprüche 1 oder 2, dadurch gekennzeichnet, dass sie etwa 0,5 bis 100 ppm Palladium bzw. Palladiumlegierung enthalten.

## Claims

1. Dental compositions which are based on polysiloxane, and can be hardened at room temperature, and which crosslink by an addition reaction and contain

a) organopolysiloxanes with two or more vinyl groups in the molecule,

b) organohydridopolysiloxanes,

c) a catalyst to accelerate the addition reaction, and

d) hydrophobic fillers and, if appropriate, other customary additives,

characterised in that

e) aluminosilicates which contain finely divided palladium and/or very finely divided palladium alloys are also added to the compositions.

2. Compositions according to claim 1, characterised in that zeolites of the faujasite type are used as the aluminosilicates.

3. Compositions according to one of claims 1 or 2, characterised in that they contain about 0.5 to 100 ppm of palladium or palladium alloy.

## Revendications

1. Matières de dentisterie à base de polysiloxannes durcissables à la température ambiante, qui se réticulent selon le système par addition, contenant

a) des organopolysiloxannes ayant deux ou plusieurs groupes vinyles dans leur molécule,

b) un organohydrogénopolysiloxanne,

c) un catalyseur pour l'accélération de la réaction d'addition, et

d) des charges hydrophobes et éventuellement d'autres additifs habituels,

caractérisées en ce que l'on ajoute en outre au matières

e) des aluminosilicates qui contiennent du palladium finement dispersé et/ou des alliages de palladium en fine dispersion.

2. Matières selon la revendication 1, caractérisées en ce que l'on utilise comme aluminosilicates des zéolites du type faujasite.

3. Matières selon l'une des revendications 1 ou 2, caractérisées en ce qu'elles contiennent environ 0,5 à 100 ppm de palladium ou d'alliage de palladium.